# EUROPEAN PATENT SPECIFICATION

(11) **EP 2 061 458 B1**
(45) Date of publication and mention of the grant of the patent: **10.12.2014**
(21) Application number: 07842475.1
(22) Date of filing: 14.09.2007
(51) Int. Cl.: A61K 31/357, A61K 31/724, A61P 25/08

(54) **TOPIRAMATE COMPOSITIONS AND METHODS FOR THEIR USE**
TOPIRAMATZUBEREITUNGEN UND VERFAHREN ZU IHRER VERWENDUNG
COMPOSITIONS DE TOPIRAMATE ET PROCÉDÉS DE LEUR UTILISATION

(30) Priority: 15.09.2006 US 844875 P
(43) Date of publication of application: 27.05.2009
(73) Proprietor: Regents of the University of Minnesota, St. Paul, Minnesota 55114-8658 (US)
(72) Inventor: CLOYD, James C., Edina, Minnesota 55424 (US)
(74) Representative: Harris, Jennifer Lucy
(86) International application number: PCT/US2007/078465
(87) International publication number: WO 2008/034040

(56) References cited:
- WO-A-2005/053612
- WO-A-2006/009403
- US-A1- 2003 235 576
- US-A1- 2005 191 343
- RAJEWSKI ET AL.: "Preliminary Safety Evaluation of Parenterally Administered Sulfoalkyl Ether beta-Cyclodextrin Derivatives" JOURNAL OF PHARMACEUTICAL SCIENCES, vol. 84, no. 8, August 1995 (1995-08), pages 927-932, XP002459611 usa
- VALENTINO J STELLA: "High Tech, Low Tech or Right Tech? The Discovery and development of a new Pharmaceutical Excipient, Captisol" YAKUZAIGAKU - JOURNAL OF PHARMACEUTICAL SCIENCE AND TECHNOLOGY, YAKUJI NIPPO-SHA, TOKYO, JP, vol. 60, no. Suppl, April 2000 (2000-04), pages 11-14, XP009092501 ISSN: 0372-7629
- MA D.Q. ET AL.: "New Injectable melphalan formulations utilizing (SBE)7m-beta-CD or HP-beta-CD" INTERNATIONAL JOURNAL OF PHARMACEUTICS, vol. 189, 1999, pages 227-234, XP002459612
- MC INTOSH M. P. ET AL.: "In Vitro and In Vivo Evaluation of a Sulfobutyl Ether beta-Cyclodextrin Enabled Etomidate Formulation" JOURNAL OF PHARMACEUTIACL SCIENCES, vol. 93, no. 10, October 2004 (2004-10), pages 2585-2594, XP002459613 USA

## Description

### Related Application(s)

This patent document claims the benefit of priority of U.S. application serial No. 60/844,875, filed September 15, 2006.

### Statement of Government Support

Work related to this patent document was funded by the U.S. government (NIH Grant NS-16308-26). The government may have certain rights in this patent document.

### Background

Topiramate (2,3:4,5-Di-O-isopropylidene-β-D-fructopysnosesulfamate) is a well-known compound that is an anticonvulsant drug used to treat epilepsy in both children and adults. It is also approved for the treatment of migraines. However, while oral formulations of topiramate are available, injectable (e.g., intravenous (IV) or intramuscular (IM)) formulations of topiramate are needed.

There is also a need for compositions and methods for providing neuroprotection and for treating neonatal seizures.

WO 2006/009403 discloses sustained-release formulations of topiramate. WO 2005/053612 discloses reverse micellar formulations for the delivery of topiramate.

### Summary of Certain Embodiments of the Invention

As described herein, compositions suitable for injectable administration that include topiramate and a cyclodextrin have been developed. These injectable compositions are useful, *e.g.,* for treating patient populations for which oral compositions of topiramate are not appropriate. For example, oral compositions of topiramate may not be appropriate because a patient may be too young, unable to swallow, undergoing GI surgery, incapacitated, or have a disorder that blocks absorption. Further, injectable compositions of topiramate would be useful for treating conditions where patients need to rapidly attain an increased concentration of topiramate. These injectable compositions also provide a more controlled dosing than do oral compositions.

Accordingly, certain embodiments of the present invention provide compositions comprising topiramate, or a salt thereof, and compound of Formula I: 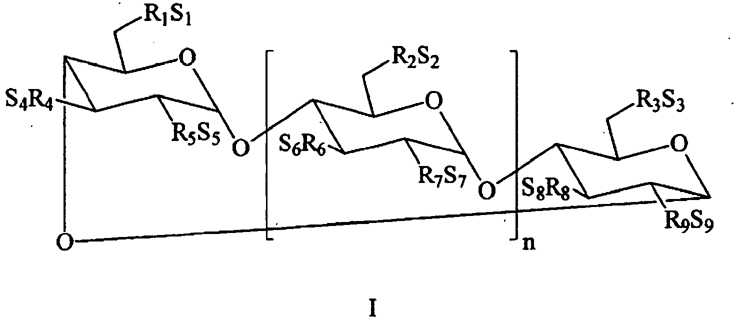 wherein:
n is 4, 5 or 6;
R₁, R₂, R₃, R₄, R₅, R₆, R₇, R₈ and R₉ are each, independently, -O- or a -O-(C₂-C₆ alkylene)-SO₃⁻ group, wherein at least one of R₁ and R₂ is independently a-O-(C₂-C₆ alkylene)-SO₃⁻ group; and
S₁, S₂, S₃, S₄, S₅, S₆, S₇, S₈ and S₉ are each, independently, H or a pharmaceutically acceptable cation; and wherein the composition is suitable for injectable administration to a patient.

Certain embodiments of the present invention provide compositions prepared by combining topiramate, or a salt thereof, and a compound of formula I: 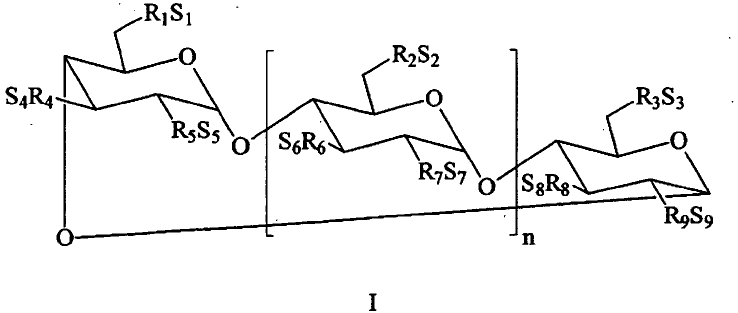 wherein:
n is 4, 5 or 6;
R₁, R₂, R₃, R₄, R₅, R₆, R₇, R₈ and R₉ are each, independently, -O- or a -O-(C₂-C₆ alkylene)-SO₃⁻ group, wherein at least one of R₁ and R₂ is independently a -O-(C₂-C₆ alkylene)-SO₃⁻ group; and
S₁, S₂, S₃, S₄, S₅, S₆, S₇, S₈ and S₉ are each, independently, H or a pharmaceutically acceptable cation; and wherein the composition is suitable for injectable administration to a patient.

In some embodiments of the invention, at least one of R₁ and R₂ is independently a -O-(C₂-C₆ alkylene)-SO₃⁻ group that is a -O-(CH₂)ₘSO₃⁻ group, wherein m is 2 to 6.

In some embodiments of the invention, the pharmaceutically acceptable cation is H, an alkali metal, an alkaline earth metal, an ammonium ion, or an amine cation.

Certain embodiments of the present invention provide compositions comprising topiramate, or a salt thereof, and a cyclodextrin such as a compound of Formula III: 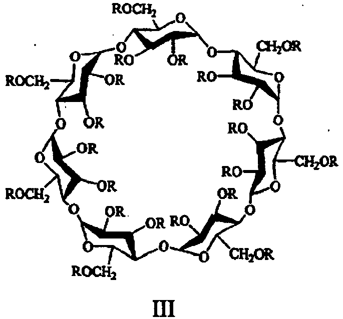 wherein R=(H)₂₁₋ₓ, or (-(CH₂)₄-SO₃Na)ₓ, and x=6.0-7.1.

Certain embodiments of the present invention provide compositions prepared by combining topiramate, or a salt thereof, and a cyclodextrin such as a compound of Formula III: 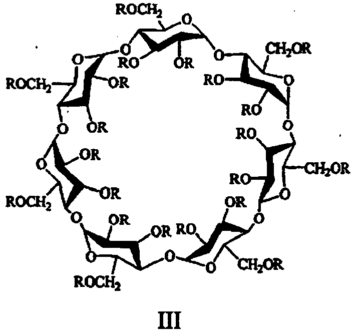 wherein R=(H)₂₁₋ₓ or (-(CH₂)₄-SO₃Na)ₓ, and x=6.0-7.1.

In some embodiments of the invention, the composition of the invention further comprises an additional therapeutic agent.

In some embodiments of the invention, the composition further comprises an additional pharmaceutically-acceptable carrier.

The composition is suitable for injectable administration to a patient. In some embodiments of the invention, the composition is suitable for intravenous or intramuscular administration to a patient.

Certain embodiments of the present invention provide compositions for use in methods for delivering topiramate to a patient, comprising administering a composition of the invention to the patient. In some embodiments of the invention, the patient is a patient in need of treatment with topiramate. In some embodiments of the invention, the composition is administered intravenously to the patient.

Certain embodiments of the present invention provide compositions for use in methods for treating a patient who has or is at risk for developing a condition amenable to treatment with topiramate comprising administering an effective amount of a composition of the invention (e.g., intravenously) to the patient so as to treat the condition.

In some embodiments of the invention, the condition is selected from epilepsy, status epilepticus, refractory status epilepticus, gambling addiction, migraines, substance dependence, alcoholism, cocaine dependence, nicotine dependence, metabolic syndrome X, diabetes mellitus, type 2, vomiting, obsessive-compulsive disorder, refractory generalized social phobia, Tourette syndrome, levodopa-induced dyskinesia in Parkinson's Disease, refractory POS, Prader-Willi syndrome, multiple sclerosis, Lennox-Gastaut syndrome, bipolar disorder, obesity, post traumatic stress disorder, cluster headaches, severe headaches, and conditions caused by exposure to a chemical warfare nerve agents such as sarin.

Certain embodiments of the present invention provide compositions for use in methods for providing neuroprotection in a patient, comprising administering an effective amount of a composition of the invention *(e.g.,* intravenously) to the patient. In some embodiments of the invention, the neuroprotection is needed during surgery.

Certain embodiments of the present invention provide composition for use in methods for treating anoxia in a patient, comprising administering an effective amount of a composition of the invention (*e.g.*, intravenously) to the patient.

Certain embodiments of the present invention provide compositions for use in methods for treating seizures in a patient, comprising administering an effective amount of a composition of the invention (*e.g*., intravenously) to the patient.

Certain embodiments of the present invention provide compositions for use in methods for loading a patient to attain an effective topiramate concentration, comprising administering an effective amount of a composition of the invention *(e.g.,* intravenously) to the patient.

In some embodiments of the invention, oral topiramate therapy for the patient has been interrupted.

In some embodiments of the invention, the patient is a neonatal patient. In some embodiments of the invention, the patient is a pediatric patient. In some embodiments of the invention, the patient is an adult patient. In some embodiments of the invention, the patient is a geriatric patient.

Certain embodiments of the present invention provide compositions of the invention for use in medical treatment or diagnosis. Certain embodiments of the present invention provide use of topiramate, or a salt thereof, and compound of Formula I: 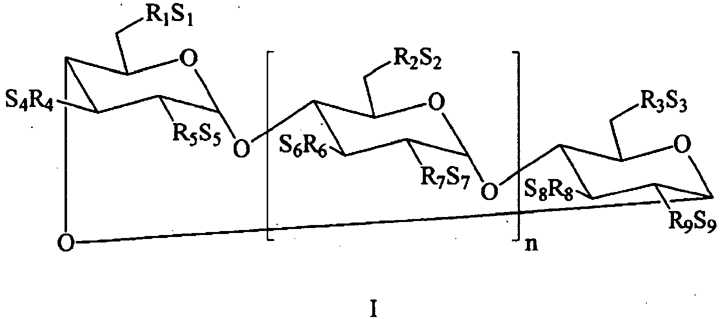 wherein:
n is 4, 5 or 6;
R₁, R₂, R₃, R₄, R₅, R₆, R₇, R₈ and R₉ are each, independently, -O- or a -O-(C₂-C₆ alkylene)-SO₃⁻ group, wherein at least one of R₁ and R₂ is independently a -O-(C₂-C₆ alkylene)-SO₃⁻ group; and
S₁, S₂, S₃, S₄, S₅, S₆, S₇, S₈ and S₉ are each, independently, a pharmaceutically acceptable cation, to prepare a medicament useful for treating a condition amenable to treatment with topiramate in an animal; and wherein the medicament is suitable for injectable administration to a patient.

Certain embodiments of the present invention provide the use of topiramate, or a salt thereof, and a cyclodextrin such as a compound of Formula III: 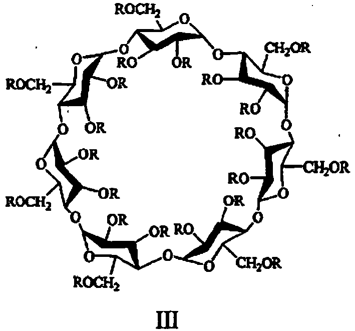 wherein R=(H)₂₁₋ₓ, or (-(CH₂)₄-SO₃Na)ₓ, and x=6.0-7.1 to prepare a medicament useful for treating a condition amenable to treatment with topiramate in an animal.

Certain embodiments of the present invention provide the use of a composition of the invention to prepare a medicament useful for treating a condition amenable to treatment with topiramate in an animal.

The medicament is suitable for injectable (e.g., intravenous) administration to a patient.

### Brief Description of the Figures

Figure 1 depicts a chromatogram from injection of a topiramate standard.
Figure 2 depicts a chromatogram from injection of a topiramate standard.
Figure 3 depicts results of a solubility study and demonstrates that topiramate is well solubilized by the cyclodextrin CAPTISOL® in water.
Figure 4 depicts results of a solubility study and demonstrates that topiramate is well solubilized by the cyclodextrin CAPTISOL® in water.
Figure 5 depicts a chromatogram for the analysis of the solubility sample using 40% w/v the cyclodextrin CAPTISOL®.

### Detailed Description

Compositions for injectable (e.g., IV) administration of topiramate and a cyclodextrin have been developed.

In certain embodiments, the composition of the invention comprises topiramate and a sulfoalkyl ether cyclodextrin of the formula I: 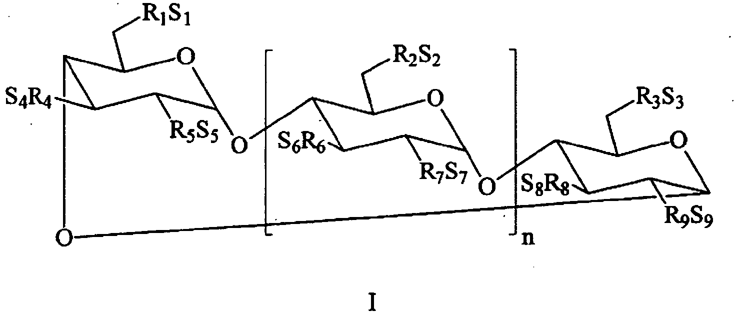 wherein:
n is 4, 5 or 6;
R₁, R₂, R₃, R₄, R₅, R₆, R₇, R₈ and R₉ are each, independently, -O- or a -O-(C₂ - C₆ alkylene)-SO₃⁻ group, wherein at least one of R₁ and R₂ is independently a -O-(C₂-C₆ alkylene)-SO₃⁻ group, preferably a -O-(CH₂)ₘSO₃⁻ group, wherein m is 2 to 6, preferably 2 to 4, (e.g.,-OCH₂CH₂CH₂SO₃⁻ or -OCH₂CH₂CH₂CH₂SO₃⁻); and
S₁, S₂, S₃, S₄, S₅, S₆, S₇, S₈ and S₉ are each, independently, H or a pharmaceutically acceptable cation which includes, for example, alkali metals *(e.g.,* Li⁺, Na⁺, K⁺), alkaline earth metals (e.g., Ca⁺², Mg⁺²), ammonium ions and amine cations such as the cations of (C₁ - C₆)-alkylamines, piperidine, pyrazine, (C₁ - C₆)-alkanolamine and (C₄ - C₈)-cycloalkanolamine; and wherein the composition is suitable for injectable administration to a patient.

In certain embodiments of the invention, the cyclodextrin is a sulfoalkyl ether cyclodextrin derivative described in U.S. Patents No. 5,134,127 or 5,376,645.

In certain embodiments of the invention, the compositions of the invention are useful for treatment of a condition amenable to treatment with topiramate, which include, *e.g.,* the treatment of epilepsy, seizures *(e.g.,* neonatal seizures), refractory status epilepticus, gambling, migraines, substance dependence, alcoholism; cocaine dependence, nicotine dependence, metabolic syndrome X; diabetes mellitus, type 2, vomiting, obsessive-compulsive disorder, refractory generalized social phobia, Tourette syndrome, levodopa-induced dyskinesia in Parkinson's Disease, refractory POS, Prader-Willi syndrome, multiple sclerosis, Lennox-Gastaut syndrome, bipolar disorder, obesity, post traumatic stress disorder, cluster headaches, severe headaches, anoxia (e.g., neonatal anoxia), and for any condition that can be treated with topiramate (e.g., for patients unable to take oral composition of topiramate).

The compositions of the invention are useful for providing neuroprotection for a patient *(e.g.,* during surgery, *e.g.,* during neonatal or pediatric surgery, *e.g.,* during heart surgery or during a stroke, head injury, or coma).

In certain embodiments, the compositions are useful for protecting brain tissue near an area of ischemic stroke (the penumbra). The compositions may be administered, *e.g.,* within a few hours after a stroke to protect the penumbra brain tissue from injury.

The compositions of the invention are also useful as a counter-measure for chemical warfare nerve agents such as sarin.

The compositions of the invention are also useful as an alternate treatment for a patient, e.g., as a bridge treatment during a period of time when a patient is not able to be treated with an oral formulation of topiramate.

The compositions of the invention are also useful for treating a patient who needs to rapidly attain or re-attain increased plasma topiramate concentrations, e.g., when those concentrations have declined as a result of not taking an oral formulation of topiramate.

The pharmaceutical dosage forms suitable for injection or infusion can include sterile aqueous solutions or dispersions or sterile powders comprising the active ingredient(s) which are adapted for the extemporaneous preparation of sterile injectable or infusible solutions or dispersions. The formulation can be provided as a stock solution, which is diluted with a liquid carrier composition such as dextrose, saline, plasma, or lactated Ringer's solution prior to administration to a patient. The formulation can be provided at a concentration of topiramate that is suitable for administration without dilution. The liquid carrier or vehicle can be a solvent or liquid dispersion medium comprising, for example, water, ethanol, a polyol (for example, glycerol, propylene glycol, liquid polyethylene glycols, and the like), vegetable oils, nontoxic glyceryl esters, and suitable mixtures thereof. The formulation can further include a preservative, a solubilizing agent, an antioxidant, a buffering agent, an acidifying agent, a complexation enhancing agent, saline, dextrose, a lyophilizing aid (for example, bulking agents or stabilizing agents), an electrolyte, another therapeutic agent, an alkalizing agent, an antimicrobial agent, an antifungal agent, an antibacterial agent (e.g., a parabens or thimersol) or a combination thereof. Prolonged absorption of the injectable compositions (e.g., by IM injection) can be brought about by the use in the compositions of agents delaying or modifying the absorption, for example, aluminum monostearate , oleaginous vehicles, less soluble salt forms, or poloxamers (block copolymers). The pharmaceutical dosage forms suitable for injection or infusion can include sterile aqueous solutions or dispersions or sterile powders of topiramate which are adapted for the extemporaneous preparation of sterile injectable or infusible solutions or dispersions, optionally encapsulated in liposomes. The ultimate dosage form should be sterile, fluid and stable under the conditions of manufacture and storage. The liquid carrier or vehicle can be a solvent or liquid dispersion medium comprising, for example, sulfoalkyl cyclodextrin in water, ethanol, a polyol (for example, glycerol, propylene glycol, liquid polyethylene glycols, and the like), vegetable oils, nontoxic glyceryl esters, and suitable mixtures thereof. The proper fluidity can be maintained, for example, by the formation of liposomes, by the maintenance of the required particle size in the case of dispersions or by the use of surfactants.

Sterile injectable solutions can be prepared by incorporating the active compound(s) into an appropriate solvent with the other optional ingredients enumerated herein, optionally followed by filter sterilization. In the case of sterile powders for the preparation of sterile injectable solutions, the preferred methods of preparation are spray drying, vacuum drying and the freeze drying techniques, which yield a powder of the active ingredient plus any additional desired ingredient present in the previously sterile-filtered solutions.

The concentration of topiramate is, typically, *e.g.,* 5-100 mg/ml, 5-50 mg/ml, 10-20 mg/ml. In some embodiments of the invention, the cyclodextrin, such as a compound of Formula III, is present at a concentration of 1-700 mg/ml.

As used herein the terms "treat", "treating" and "treatment" include administering the composition prior to the onset of clinical symptoms of a disease state/condition so as to prevent the development of any symptom, as well as administering the composition after the onset of one or more clinical symptoms of a disease state/condition so as to reduce or eliminate any such symptom, aspect or characteristic of the disease state/condition. Such treating need not be absolute to be useful.

The compositions may, in certain embodiments, be provided in a unit dosage form or in a container from which a dose is measured out. As used herein the term "unit dosage form" relates to a composition containing a specific amount of a drug, the whole of which is intended to be administered as a single dose. It is distinguished from a supply of a multi-dose amount of a medicament, *e.g.,* a bottle of medicine, from which a dose has to be measured out.

As used herein, the term "patient" is taken to mean warm blooded animals such as mammals, for example, cats, dogs, mice, guinea pigs, horses, bovine cows, sheep, and humans.

In certain embodiments of the invention, treatment may include multiple doses, *e.g.,* doses occurring over days, weeks, or years.

The compositions may also include at least one additional therapeutic agent.

In certain embodiments of the invention, the compositions of the invention can be administered to neonatal, pediatric, adult, or geriatric patients. Neonatal patients are of about 0-30 days of age. Pediatric patients are of about up to 17 years of age. Adult patients are at least about 18 years of age. Geriatric patients are at least about 65 years of age.

### Topiramate

The compositions of the invention include topiramate (see, e.g., U.S. Patent Nos. 6,949,518, 6,906,099, 6,699,840, 6,696,091 6,559,293, 6,503,884, 5,952,187, 5,258,402, and 4,513,006). Methods for preparing topiramate are known in the art. Topiramate is designated chemically as 2,3:4,5-Di-O-isopropylidene-β-D-fructopyranosesulfamate and has the following formula II:

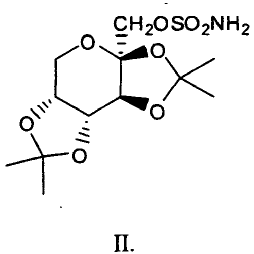

### The cyclodextrin

The compositions of the invention also include a cyclodextrin molecule *(e.g.,* a sulfobutyl ether-ß-cyclodextrin such as CAPTISOL®, see, *e.g.,* U.S. Patent Nos. 6,133,248, 5,874,418, 6,046,177, 5,376,645, 5,134,127, 7,034,013, 6,869,939, WO 2005/117911 and MSDS Number CAP-001). Methods for preparing a sulfobutyl ether-ß-cyclodextrin are known in the art. The compositions containing the sulfoalkyl ether cyclodextrin will have improved solubility, stability and/or bioavailability of topiramate.

CAPTISOL® cyclodextrin is a modified cyclodextrin. CAPTISOL® cyclodextrin is a polyanionic beta-cyclodextrin derivative with a sodium sulfonate salt separated from the lipophilic cavity by a butyl ether spacer group, or sulfobutylether (SBE). CAPTISOL® cyclodextrin has been shown to be safe when administered parenterally, orally and via inhalation and does not exhibit the nephrotoxicity associated with beta-cyclodextrin. Relative to beta-cyclodextrin, CAPTISOL® cyclodextrin provides comparable or higher complexation characteristics and superior water solubility in excess of 90 grams/100ml, a 50-fold improvement. CAPTISOL® cyclodextrin has the following forumula III: 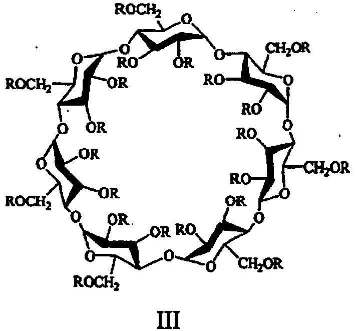 where R=(H)₂₁₋ₓ or (-(CH₂)₄-SO₃Na)ₓ and, x=6.0-7.1.

In some embodiments of the invention, the cyclodextrin to topiramate mole ratio is about 0.01 to 1.4.

The invention will now be illustrated by the following non-limiting Example.

### Example 1 - Topiramate Phase Solubility Study

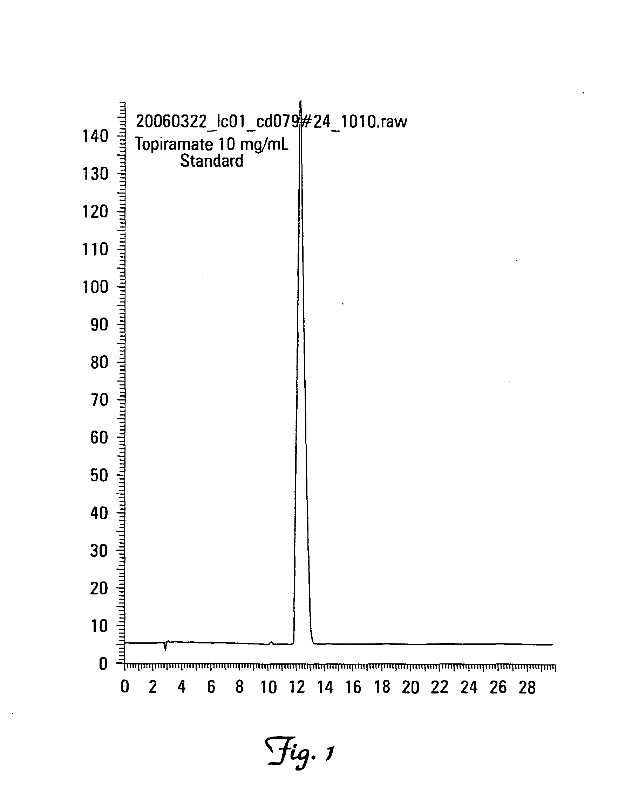

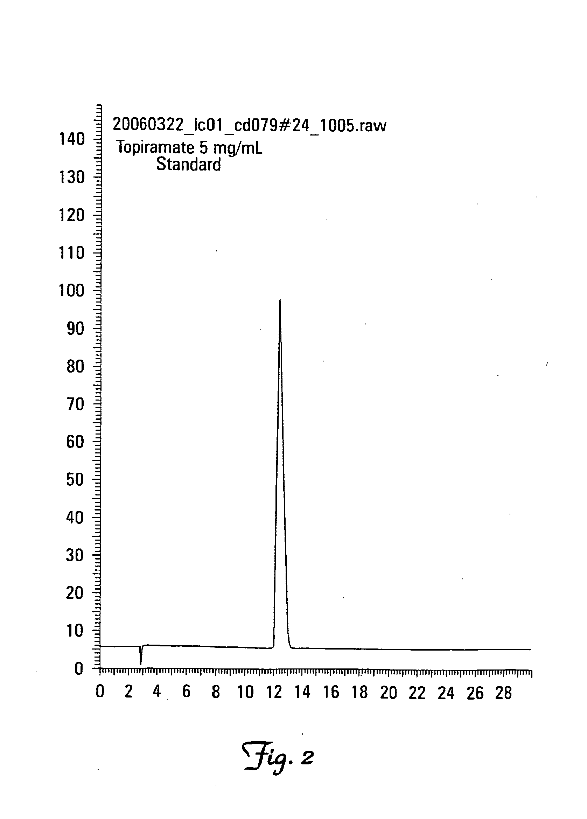

A phase solubility study was conducted with the cyclodextrin CAPTISOL® and topiramate to evaluate the extent of solubilization of the drug by the derivatized cyclodextrin. An HPLC method was modified from the literature and shown to be linear over the range of interest. Chromatograms from injection of two of the topiramate standards are shown in Figures 1 and 2.

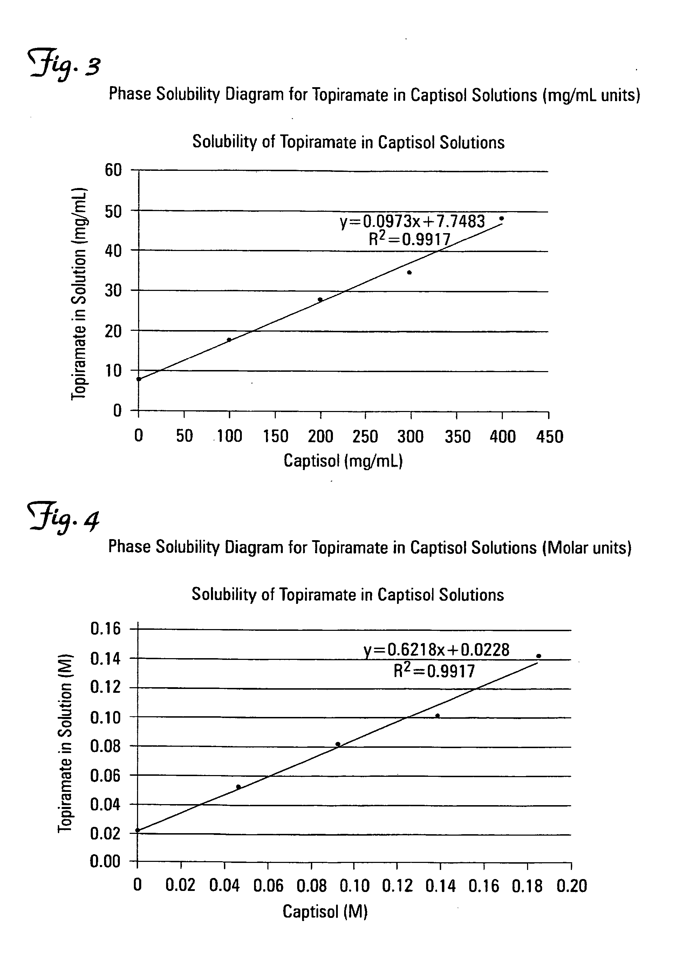

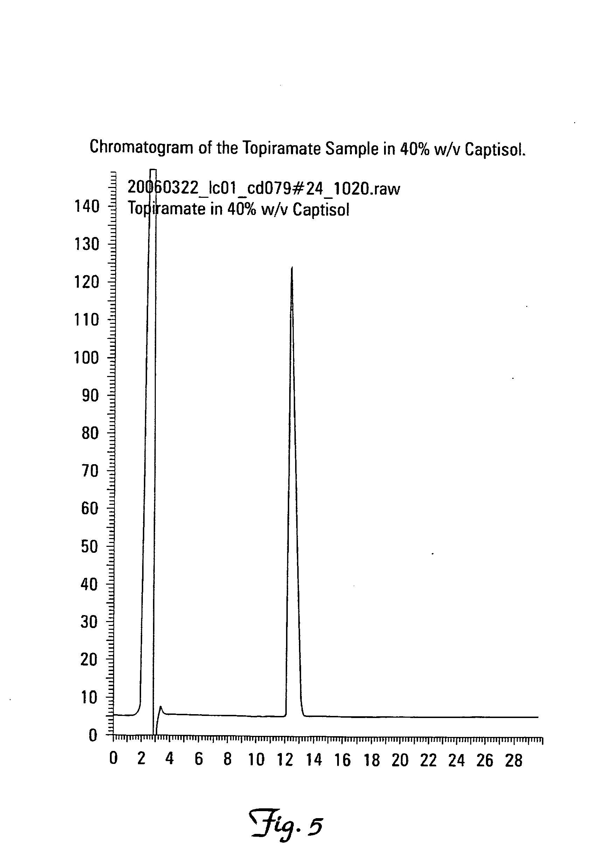

Results of the solubility study are illustrated in Figures 3 and 4 and show that topiramate is well solubilized by the cyclodextrin CAPTISOL® in water. Type A-linear phase solubility is observed and a binding constant of 71 M⁻¹ was calculated from the equation: K_{1:1} = slope/Sₒ(1-slope), where Sₒ is the intrinsic solubility of the drug and "slope" is the slope of the molar plot of drug solubility vs cyclodextrin content. The magnitude of the calculated binding constant is low due to the drug being reasonably soluble in water in the absence of cyclodextrin (intrinsic solubility of 7.86 mg/mL). A chromatogram for the analysis of the solubility sample using 40% w/v of the cyclodextrin CAPTISOL® is given in Figure 5.

### Methods:

Solutions containing increasing amounts of dissolved CAPTISOL® brand of sulfobutylether b-cyclodextrin were prepared and added to small glass vials. Excess solid topiramate was added to each vial and the vials were capped, vortexed and placed in constant agitation for five days at room temperature (∼23-25°C). If any vial showed complete dissolution of the added drug, additional drug was added and the vial returned to the stirring mode.

After the multiday equilibration period, the vials were centrifuged (twice at 693 x g, 25°C) and aliquots were taken from the clear supernatant solutions. The aliquots were diluted 1:3 (1:5.67 for 40% cyclodextrin CAPTISOL® solutions) with mobile phase and analyzed by HPLC for topiramate content.

### Materials:

Topiramate: Lot #LL-001-009-III-01 (Divi's Laboratories Ltd, Ameerpet, Hyderabad 500016, India)

The cyclodextrin CAPTISOL®: Lot #17CX01.HQ00009 (CyDex, Inc. Lenexa, Ks)

### Chromatography:

### Chromatographic Conditions

| | |
|---|---|
| HPLC: | Dionex |
| Detection: | Refractive Index Detector |
| Column: | SB-Phenyl (5 µm) 250 mm x 4.6 mm |
| Column Temperature: | 35°C |
| Mobile Phase: | 40:60 MeOH: Water |
| Flow Rate: | 1.0 mL/min isocratic |
| Run Time: | 30 minutes |
| Sample Solvent: | Mobile phase |
| Injection Volume: | 50 µL |
| Retention Time: | ∼12.5 minutes |

### Mobile Phase Preparation

Combined 400 mL of methanol and 600 mL of water, mixed well and filtered.

### Standard Solutions

1 -TPM1 0 Solution: ∼250 mg of TPM was weighed into a 25 mL volumetric flask, diluted to volume with mobile phase, and mixed well.
2-TPM5 Solution: 5 mL of TPM1O solution were transferred into a 10 mL volumetric flask and diluted to volume with mobile phase.
3-TPM1 Solution: 1 mL of TPMlO solution was transferred into a 10 mL volumetric flask and diluted to volume with mobile phase.

While in the foregoing specification this invention has been described in relation to certain embodiments thereof, and many details have been set forth for purposes of illustration, it will be apparent to those skilled in the art that the invention is susceptible to additional embodiments and that certain of the details described herein may be varied considerably without departing from the basic principles of the invention.

## Claims

1. A composition comprising topiramate, or a salt thereof, and compound of Formula I: 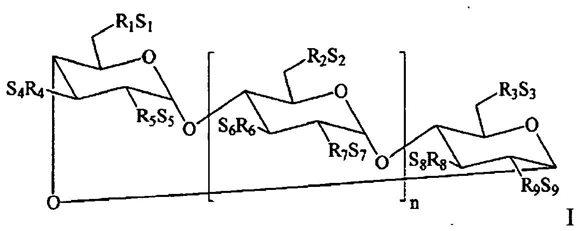 wherein:
n is 4, 5 or 6;
R₁, R₂, R₃, R₄, R₅, R₆, R₇, R₈ and R₉ are each, independently, -O- or a -O-(C₂-C₆ alkylene)-SO₃⁻ group, wherein at least one of R₁ and R₂ is independently a -O-(C₂-C₆ alkylene)-SO₃⁻ group; and
S₁, S₂, S₃, S₄, S₅, S₆, S₇, S₈ and S₉ are each, independently, H or a pharmaceutically acceptable cation; and wherein the composition is suitable for injectable administration to a patient.

2. The composition of claim 1, wherein at least one of R₁ and R₂ is independently a -O-(C₂-C₆ alkylene)-SO₃⁻ group that is a -O-(CH₂)ₘSO₃⁻ group, wherein m is 2 to 6, and the pharmaceutically acceptable cation is H, an alkali metal, an alkaline earth metal, an ammonium ion, or an amine cation.

3. The composition of claim 1, wherein the compound of Formula I is a compound of Formula III: 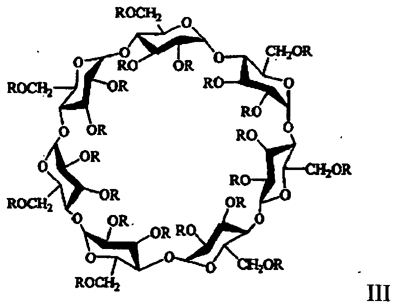 wherein R=(H)₂₁₋ₓ or (-(CH₂)₄-SO₃Na)ₓ, wherein x = 6.0-7.1.

4. The composition of any one of claims 1-3, wherein the composition further comprises an additional therapeutic agent, and/or a pharmaceutically-acceptable carrier.

5. The composition of any one of claims 1-4, wherein the topiramate is present in the composition at a concentration of 5-100 mg/ml, and/or wherein the compound of Formula I is present in the composition at a concentration of 1-700 mg/ml.

6. The composition of any one of claims 1-5, wherein the topiramate is present in the composition at a concentration of 5-50 mg/ml.

7. The composition of any one of claims 1-6, wherein the topiramate is present in the composition at a concentration of 10-20 mg/ml.

8. The composition of any one of claims 1-7, wherein the composition is suitable for intravenous administration to a patient.

9. The composition of any one of claims 1-8 for delivering topiramate to a patient.

10. An effective amount of the composition of any one of claims 1-8 for use in treating a patient who has or is at risk for developing a condition amenable to treatment with topiramate, wherein the composition is intravenously administered to the patient so as to treat the condition, and wherein the condition is selected from epilepsy, status epilepticus, refractory status epilepticus, gambling addiction, migraines, substance dependence, alcoholism; cocaine dependence, nicotine dependence, metabolic syndrome X; diabetes mellitus, type 2, vomiting, obsessive- compulsive disorder, refractory generalized social phobia, Tourette syndrome, levodopa-induced dyskinesia in Parkinson's Disease, refractory POS, Prader- Willi syndrome, multiple sclerosis, Lennox-Gastaut syndrome, bipolar disorder, obesity, post traumatic stress disorder, cluster headaches, severe headaches, and conditions caused by exposure to a chemical warfare nerve agent.

11. An effective amount of the composition of any one of claims 1-8 for use in providing neuroprotection in a patient, wherein the composition is administered intravenously to the patient.

12. An effective amount of the composition of any one of claims 1-8 for use according to claim 11, wherein the neuroprotection is needed during surgery.

13. An effective amount of the composition of any one of claims 1-8 for use in treating anoxia in a patient, for use in treating seizures in a patient, for use in treating a stroke in a patient, and/or for use in loading a patient to attain an effective topiramate concentration, wherein the composition is administered intravenously to the patient.

14. An effective amount of the composition of any one of claims 1-8 for use according to claim 13 in loading a patient to attain an effective topiramate concentration, wherein oral topiramate therapy for the patient has been interrupted.

15. An effective amount of the composition of any one of claims 1-8 for use according to any one of claims 9-14, wherein the patient is a neonatal patient, a pediatric patient, an adult patient, and/or a geriatric patient.

16. A composition as described by any one of claims 1-8 for use in medical treatment or diagnosis.

## Patentansprüche

1. Zusammensetzung, umfassend Topiramat oder ein Salz davon und die Verbindung der Formel I: 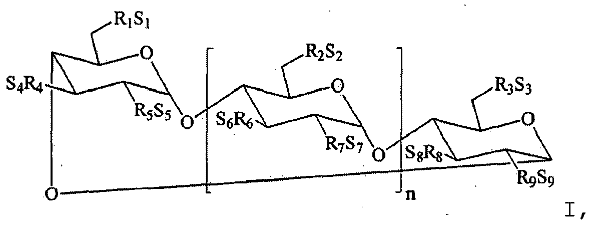 wobei:
n für 4, 5 oder 6 steht;
R₁, R₂, R₃, R₄, R₅, R₆, R₇, R₈ und R₉ jeweils unabhängig voneinander für -0- oder eine -O-(C₂-C₆-Alkylen)-SO₃⁻-Gruppe stehen, wobei mindestens einer der Reste R₁ und R₂ unabhängig von dem anderen für eine -O-(C₂-C₆-Alkylen)-SO₃⁻-Gruppe steht;
S₁, S₂, S₃, S₄, S₅, S₆, S₇, S₈ und S₉ jeweils unabhängig voneinander für H oder ein pharmazeutisch unbedenkliches Kation stehen; und
wobei sich die Zusammensetzung für die Verabreichung durch Injektion an einen Patienten eignet.

2. Zusammensetzung nach Anspruch 1, wobei mindestens einer der Reste R₁ und R₂ unabhängig von dem anderen für eine -0- (C₂-C₆-Alkylen) -SO₃⁻-Gruppe steht, bei der es sich um eine -0-(CH₂)ₘSO₃⁻-Gruppe handelt, in welcher m für 2 bis 6 steht, und es sich bei dem pharmazeutisch unbedenklichen Kation um H, ein Alkalimetall, ein Erdalkalimetall, ein Ammoniumion oder ein Aminkation handelt.

3. Zusammensetzung nach Anspruch 1, wobei es sich bei der Verbindung der Formel I um eine Verbindung der Formel III handelt: 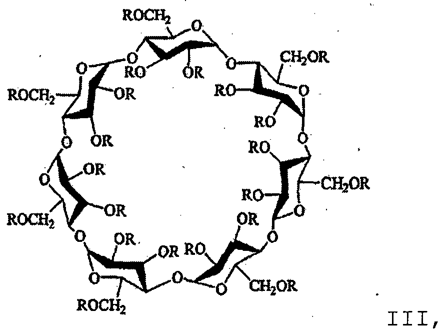 wobei R=(H)₂₁₋ₓ oder (-(CH₂)₄-SO₃Na)ₓ, wobei x = 6, 0-7,1.

4. Zusammensetzung nach einem der Ansprüche 1-3, wobei die Zusammensetzung weiterhin ein zusätzliches therapeutisches Mittel und/oder einen pharmazeutisch unbedenklichen Träger umfasst.

5. Zusammensetzung nach einem der Ansprüche 1-4, wobei das Topiramat in der Zusammensetzung in einer Konzentration von 5-100 mg/ml vorliegt und/oder wobei die Verbindung der Formel I in der Zusammensetzung in einer Konzentration von 1-700 mg/ml vorliegt.

6. Zusammensetzung nach einem der Ansprüche 1-5, wobei das Topiramat in der Zusammensetzung in einer Konzentration von 5-50 mg/ml vorliegt.

7. Zusammensetzung nach einem der Ansprüche 1-6, wobei das Topiramat in der Zusammensetzung in einer Konzentration von 10-20 mg/ml vorliegt.

8. Zusammensetzung nach einem der Ansprüche 1-7, wobei sich die Zusammensetzung für die intravenöse Verabreichung an einen Patienten eignet.

9. Zusammensetzung nach einem der Ansprüche 1-8 zur Verabreichung von Topiramat an einen Patienten.

10. Wirksame Menge der Zusammensetzung nach einem der Ansprüche 1-8 zur Verwendung bei der Behandlung eines Patienten, der ein Leiden hat, das einer Behandlung mit Topiramat zugänglich ist, oder bei dem ein Risiko eines Auftretens eines solchen Leidens besteht, wobei die Zusammensetzung dem Patienten zur Behandlung des Leidens intravenös verabreicht wird und wobei das Leiden ausgewählt ist aus Epilepsie, Status epilepticus, refraktärem Status epilepticus, Spielsucht, Migräne, Substanzabhängigkeit, Alkoholismus, Kokainabhängigkeit, Nikotinabhängigkeit, metabolischem Syndrom X, Diabetes mellitus vom Typ 2, Erbrechen, Zwangsneurose, refraktärer generalisierter sozialer Phobie, Tourette-Syndrom, durch Levodopa induzierte Dyskinesie bei der Parkinson-Krankheit, refraktärer POS, Prader-Willi-Syndrom, multipler Sklerose, Lennox-Gastaut-Syndrom, bipolarer Störung, Obesitas, akuter Belastungsreaktion, Cluster Headaches, schweren Kopfschmerzen und Zuständen, die durch Exposition gegenüber einem chemischen Nervenkampfstoff verursacht werden.

11. Wirksame Menge der Zusammensetzung nach einem der Ansprüche 1-8 zur Verwendung bei der Bereitstellung von Neuroprotektion bei einem Patienten, wobei die Zusammensetzung dem Patienten intravenös verabreicht wird.

12. Wirksame Menge der Zusammensetzung nach einem der Ansprüche 1-8 zur Verwendung nach Anspruch 11, wobei die Neuroprotektion während eines operativen Eingriffs benötigt wird.

13. Wirksame Menge der Zusammensetzung nach einem der Ansprüche 1-8 zur Verwendung bei der Behandlung von Anoxie bei einem Patienten, zur Verwendung bei der Behandlung von Krampfanfällen bei einem Patienten, zur Verwendung bei der Behandlung eines Schlaganfalls bei einem Patienten und/oder zur Verwendung bei der Beladung eines Patienten zum Erzielen einer wirksamen Topiramatkonzentration, wobei die Zusammensetzung dem Patienten intravenös verabreicht wird.

14. Wirksame Menge der Zusammensetzung nach einem der Ansprüche 1-8 zur Verwendung nach Anspruch 13 bei der Beladung eines Patienten zum Erzielen einer wirksamen Topiramatkonzentration, wobei eine orale Topiramattherapie des Patienten unterbrochen wurde.

15. Wirksame Menge der Zusammensetzung nach einem der Ansprüche 1-8 zur Verwendung nach einem der Ansprüche 9-14, wobei es sich bei dem Patienten um einen neugeborenen Patienten, einen pädiatrischen Patienten, einen erwachsenen Patienten und/oder einen geriatrischen Patienten handelt.

16. Zusammensetzung nach einem der Ansprüche 1-8 zur Verwendung bei der medizinischen Behandlung oder Diagnose.

## Revendications

1. Composition comprenant du topiramate, ou un sel de ce dernier, et un composé de Formule I : 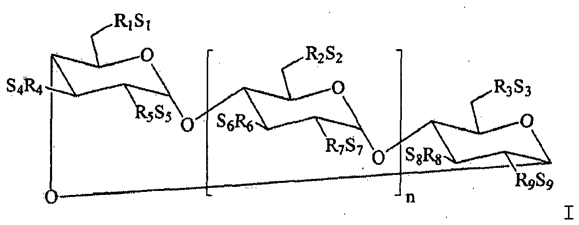 où :
n est égal à 4, 5 ou 6 ;
chacun des radicaux R₁, R₂, R₃, R₄, R₅, R₆, R₇, R₈ et R₉ représente indépendamment -0- ou un groupement -0-(alkylène en C₂-C₆) -SO₃⁻, au moins l'un des radicaux R₁ et R₂ représentant indépendamment un groupement -0-(alkylène C₂-C₆)-SO₃⁻ ; et
chacun des radicaux S₁, S₂, S₃, S₄, S₅, S₆, S₇, S₈ et S₉ représente indépendamment H ou un cation pharmaceutiquement acceptable ; et la composition étant adaptée à une administration par injection à un patient.

2. Composition selon la revendication 1, où au moins l'un des radicaux R₁ et R₂ représente indépendamment un groupement -0-(alkylène en C₂-C₆)-SO₃⁻ qui est un groupement -O-(CH₂)ₘSO₃⁻, où m est compris entre 2 et 6, et le cation pharmaceutiquement acceptable est H, un métal alcalin, un métal alcalino-terreux, un ion ammonium ou un cation d'amine.

3. Composition selon la revendication 1, où le composé de Formule 1 est un composé de Formule III : 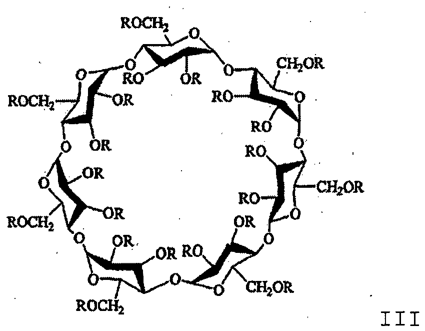 où R = (H)₂₁₋ₓ ou (-(CH₂)₄-SO₃Na)ₓ, où x = 6,0 - 7,1.

4. Composition selon l'une quelconque des revendications 1 à 3, où la composition comprend en outre un agent thérapeutique supplémentaire, et/ou un vecteur pharmaceutiquement acceptable.

5. Composition selon l'une quelconque des revendications 1 à 4, où le topiramate est présent dans la composition à une concentration comprise entre 5 et 100 mg/ml, et/ou le composé de Formule I est présent dans la composition à une concentration comprise entre 1 et 700 mg/ml.

6. Composition selon l'une quelconque des revendications 1 à 5, où le topiramate est présent dans la composition à une concentration comprise entre 5 et 50 mg/ml.

7. Composition selon l'une quelconque des revendications 1 à 6, où le topiramate est présent dans la composition à une concentration comprise entre 10 et 20 mg/ml.

8. Composition selon l'une quelconque des revendications 1 à 7, où la composition est adaptée à une administration intraveineuse à un patient.

9. Composition selon l'une quelconque des revendications 1 à 8 pour la libération de topiramate chez un patient.

10. Quantité active de la composition selon l'une quelconque des revendications 1 à 8 pour utilisation dans le traitement d'un patient qui souffre ou risque de développer un état pathologique susceptible d'être traité par le topiramate, la composition étant administrée par voie intraveineuse au patient de sorte à traiter l'état pathologique, et l'état pathologique étant choisi parmi les suivants : épilepsie, état de mal épileptique, état de mal épileptique réfractaire, addiction aux jeux d'argent, migraines, dépendance à une substance, alcoolisme ; dépendance à la cocaïne, dépendance à la nicotine, syndrome métabolique ; diabète sucré de type 2, vomissements, trouble obsessionnel compulsif, phobie sociale généralisée réfractaire, syndrome de Gilles de la de Tourette, dyskinésie induite par la levodopa dans la maladie de Parkinson, crises d'épilepsie partielle réfractaires, syndrome de Prader-Willi, sclérose en plaques, syndrome de Lennox-Gastaut, trouble bipolaire, obésité, troubles de stress post-traumatique, céphalées vasculaires de Horton, céphalées sévères, et états pathologiques provoqués par l'exposition à une arme chimique agissant sur le système nerveux.

11. Quantité active de la composition selon l'une quelconque des revendications 1 à 8 pour utilisation dans l'obtention d'une neuroprotection chez un patient, où la composition est administrée par voie intraveineuse au patient.

12. Quantité active de la composition selon l'une quelconque des revendications 1 à 8 pour utilisation selon la revendication 11, où la neuroprotection est nécessaire pendant la chirurgie.

13. Quantité active de la composition selon l'une quelconque des revendications 1 à 8 pour utilisation dans le traitement de l'anoxie chez un patient, pour utilisation dans le traitement des crises d'épilepsie chez un patient, pour utilisation dans le traitement d'un accident vasculaire cérébral chez un patient, et/ou pour utilisation dans l'administration à un patient dans le but d'atteindre une concentration active en topiramate, la composition étant administrée au patient par voie intraveineuse.

14. Quantité active de la composition selon l'une quelconque des revendications 1 à 8 pour utilisation selon la revendication 13 dans l'administration à un patient dans le but d'atteindre une concentration active en topiramate, où une thérapie orale à base de topiramate pour le patient a été interrompue.

15. Quantité active de la composition selon l'une quelconque des revendications 1 à 8 pour utilisation selon l'une quelconque des revendications 9 à 14, où le patient est un patient néonatal, un patient pédiatrique, un patient adulte et/ou un patient gériatrique.

16. Composition selon l'une quelconque des revendications 1 à 8 pour utilisation en traitement ou diagnostic médical.
